(19) Europäisches Patentamt
European Patent Office
Office européen des brevets

(11) **EP 3 424 445 B1**

(12) **EUROPÄISCHE PATENTSCHRIFT**

(45) Veröffentlichungstag und Bekanntmachung des Hinweises auf die Patenterteilung:
**27.11.2019 Patentblatt 2019/48**

(51) Int Cl.:
**A61B 17/225** (2006.01)     **H01F 27/32** (2006.01)
**H01F 38/42** (2006.01)

(21) Anmeldenummer: **18181315.5**

(22) Anmeldetag: **03.07.2018**

(54) **SCHALLWELLENBEHANDLUNGSGERÄT**

ACOUSTIC WAVE TREATMENT APPARATUS

APPAREIL DE TRAITEMENT AUX ONDES SONORES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.07.2017   DE 102017211400**

(43) Veröffentlichungstag der Anmeldung:
**09.01.2019   Patentblatt 2019/02**

(73) Patentinhaber: **Richard Wolf GmbH**
**75438 Knittlingen (DE)**

(72) Erfinder:
• **Valet, Dominic**
**76703 Kraichtal (DE)**
• **Wössner, Alexander**
**76703 Kraichtal (DE)**

(74) Vertreter: **Patentanwälte Vollmann Hemmer Lindfeld**
**Partnerschaft mbB**
**Wallstraße 33a**
**23560 Lübeck (DE)**

(56) Entgegenhaltungen:
**EP-A1- 1 163 884     CN-Y- 201 259 819**
**DE-A1- 3 432 812     US-A- 4 408 176**
**US-A- 5 543 831       US-A1- 2012 203 297**

EP 3 424 445 B1

**Beschreibung**

Diese Offenbarung betrifft ein Schallwellenbehandlungsgerät für die medizinische Behandlung mit Druckwellen, insbesondere zur Lithotripsie.

Schallwellenbehandlungsgeräte werden heutzutage in der Medizin zahlreich angewendet, und zwar nicht nur zur Stoßwellentherapie zum Zwecke der Steinzertrümmerung (Lithotripsie), sondern auch zu zahlreichen anderen Anwendungen, wie beispielsweise zur nichtinvasiven Behandlung des knochennahen Weichteilbereichs oder zur großflächigen Behandlung von beispielsweise Muskelverspannungen, Muskelverhärtungen, Bindegewebsverdichtungen oder auch zur Behandlung von großflächigen Weichteilregionen wie Fett- und Hautgewebe, Cellulitis, chronische Wunden, Entzündungsherde und dergleichen.

Bei der extrakorporalen Schallwellen-Lithotripsie (ESWL) werden Schallwellen durch sphärisch angeordnete und mit Hochspannung über Entladekondensatoren und Thyristorschalter konzertiert angeregte Piezoelemente erzeugt, wobei sich die Schallwellen in einer Fokuszone zu einem lokal sehr hohen Druck (zum Teil mehr als 100 MPa) überlagern. Die Fokuszone ist dabei beispielsweise gezielt auf einen im Körper eines Patienten befindlichen Harn- oder Nierenstein gerichtet, so dass der Stein durch den Druck aufgesprengt wird. Die DE 10 2010 055 836 B4 beschreibt bespielweise solch eine piezoelektrische Stoßwellenquelle, die wie in der DE 44 33 224 C1 beschrieben angesteuert werden kann.

Da derartige ESWL-Geräte mit Hochspannung arbeiten, werden hohe Sicherheitsanforderungen an den Patienten- und Anwenderschutz gestellt. Gemäß der dritten Ausgabe der internationalen Norm IEC 60601-1, die Wechselspannungen oberhalb von 1 kV und Gleichspannungen oberhalb von 1,5 kV als Hochspannung bezeichnet, werden für medizinische Geräteteile mit Patientenkontakt zwei unabhängige Schutzmaßnahmen zum Patientenschutz (engl.: means of patient protection (MOPP)) gefordert. Für Geräteteile mit Anwenderkontakt, aber ohne Patientenkontakt, werden zwei unabhängige Schutzmaßnahmen zum Anwenderschutz (engl.: means of operator protection (MOOP)) gefordert. Zusätzlich wird eine Erstfehlersicherheit gefordert, was bedeutet, dass bei erstmaligem Auftritt eines Fehlers keine Gefährdung für den Patienten oder Anwender besteht.

Bei einer Verringerung des Bauraums für die Hochspannungsversorgung solch eines ESWL-Geräts muss daher eine hohe Isolationsfestigkeit und die Erstfehlersicherheit gewährleistet bleiben.

Die EP 1 163 884 A1 beschreibt eine Vorrichtung zum Entfernen von Körpersteinen mit einem intrakorporalen Lithotripter.

Die US 4,408,176 Beschreibt einen Flyback-Transformator zur Verwendung in einem TV-Receiver.

Die US 2012/0203297 A1 beschreibt ein Defibrillationsgerät zur Stimulation der Herzmuskeln.

Das Schallwellenbehandlungsgerät gemäß Anspruch 1 der vorliegenden Offenbarung hat dagegen bei kleinerer Baugröße eine hohe Isolationsfestigkeit und ist erstfehlersicher. Vorteilhafte Ausgestaltungen des hierin offenbarten Schallwellenbehandlungsgeräts sind den Unteransprüchen, der nachfolgenden Beschreibung und der Zeichnung zu entnehmen.

Das hierin offenbarte Schallwellenbehandlungsgerät für die medizinische Behandlung mit Druckwellen, insbesondere zur Lithotripsie, weist einen Schallwellenerzeuger, eine Mehrzahl von Piezoelementen, die mit dem Schallwellenerzeuger gekoppelt sind, und eine elektrische Hochspannungseinheit zur Versorgung der Piezoelemente mit elektrischer Hochspannung auf. Die Hochspannungseinheit weist dabei eine Sperrwandlereinheit mit einem Transformator auf. Der Transformator weist dabei eine Primärwicklung, eine Hochspannungswicklung und Abstandsmittel auf. Die Hochspannungswicklung hat eine Mehrzahl von Hochspannungswindungen, die, vorzugsweise im Wesentlichen luftblasenfrei, in eine Isolationsmasse eingebettet sind und durch die Abstandsmittel derart positioniert sind, dass benachbarte Hochspannungswindungen einen durch die Abstandsmittel definierten Abstand voneinander haben.

Die Sperrwandlereinheit in der Hochspannungseinheit hat den Vorteil, dass die Hochspannungsseite des Transformators (Sekundärseite) galvanisch von der Eingangsspannungsseite des Transformators (Primärseite) getrennt ist, wodurch die Erstfehlersicherheit erhöht wird. Die vorzugsweise im Wesentlichen luftblasenfreie Einbettung in eine Isolationsmasse im Zusammenspiel mit den Abstandsmitteln erhöht die Isolationsfestigkeit und erlaubt zugleich eine kleinere Baugröße des Transformators. Simulationen und Testversuche haben gezeigt, dass das hierin offenbarte Schallwellenbehandlungsgerät zudem kürzere Ladezeiten der Entladekondensatoren hat, sodass das Schallwellenbehandlungsgerät effizienter eingesetzt werden kann.

Optional kann die Hochspannungswicklung auf einem Hochspannungsspulenformer gewickelt sein und die Abstandsmittel können radial aus dem Hochspannungsspulenformer zwischen benachbarte Hochspannungswindungen ragen. Die zwischen benachbarten Hochspannungswindungen maximal auftretenden Feldstärken werden durch die dazwischen ragenden Abstandsmittel reduziert, wodurch der Transformator isolationsfester ist.

Optional kann die Hochspannungswicklung auf einem Hochspannungsspulenformer gewickelt sein und die Hochspannungswindungen können in einer auf einer äußeren Mantelfläche des Hochspannungsspulenformers verlaufenden Nut verlaufen. Dies macht das Vorsehen der Abstandsmittel besonders einfach, da sie durch den Hochspannungsspulenformer selbst gebildet sein können.

Optional können die Abstandsmittel und/oder die Nut Zentrierflächen aufweisen, die die Hochspannungswindungen in eine eindeutige Lage zwingt. Da die Isolationsmasse beispielweise als Vergussmasse über die Hochspannungswindungen gegossen wird, können die Zentrierflächen einen genauen und sich nicht beim Vergießen ändernden Abstand benachbarter Hochspannungswindungen sicherstellen. Außerdem werden durch den definierten Abstand Luftblaseneinschlüsse beim Vergießen vermieden. Unerwünschte Luftblasen in der Isolationsmasse könnten nämlich lokal zu hohe Feldstärken zulassen, was die Isolationsfestigkeit beeinträchtigt. Die Zentrierflächen können beispielsweise durch die schrägen Schenkelflächen einer V-förmigen oder U-förmigen Nut gebildet sein.

Optional kann die Hochspannungswicklung auf einen Hochspannungsspulenformer gewickelt und das Abstandsmittel ein Gewinde auf dem Hochspannungsspulenformer sein. Das Gewinde kann dabei eine helixförmig um den Hochspannungsspulenformer umlaufende Nut definieren, wobei die Gewindestege zwischen der Nut die Abstandsmittel bilden. Das Vorsehen von Abstandsmitteln in Form eines Gewindes auf dem Hochspannungsspulenformer ist wenig komplex und erlaubt eine kleine Baugröße des Transformators.

Optional kann der Transformator eine erste Mantelfläche mit einem ersten Radius und eine zweite Mantelfläche mit einem zweiten Radius aufweisen, wobei die Primärwicklung auf der ersten Mantelfläche und die Hochspannungswicklung auf der zweiten Mantelfläche gewickelt ist, wobei der erste Radius anders, vorzugsweise kleiner, ist als der zweite Radius. Durch die Mantelflächen mit unterschiedlichem Radius werden magnetische Kopplungen und daraus resultierende mechanische Spannungen zwischen der Primärwicklung und der Hochspannungswicklung verringert.

Optional kann die Primärwicklung zumindest teilweise innerhalb der Hochspannungswicklung verlaufen. Optional kann dazu die Hochspannungswicklung auf einem Hochspannungsspulenformer und die Primärwicklung auf einem Primärspulenformer gewickelt sein, wobei der Hochspannungsspulenformer den Primärspulenformer koaxial umgreifen kann. Damit sind ein Zusammenbau des Transformators und ein späteres Vergießen der Isolationsmasse um die Hochspannungswicklung besonders einfach.

Optional kann der Transformator eine erste Hilfswicklung zur indirekten Messung der ausgangsseitigen Hochspannung aufweisen. Die Hilfswicklung kann dabei galvanisch getrennt von der Hochspannung dazu verwendet werden, die Hochspannung zu messen, damit diese geregelt oder im Fehlerfall als Alarm angezeigt bzw. abgeschaltet werden kann. Damit trägt die Hilfswicklung zur Erstfehlersicherheit bei, ohne über eine galvanische Kopplung mit der Hochspannung eine weitere Fehlerquelle einzuführen.

Optional kann der Transformator eine zweite Hilfswicklung zur redundanten indirekten Messung der ausgangsseitigen Hochspannung aufweisen, die parallel zur ersten Hilfswicklung angeordnet und geschaltet ist. Bei einem Versagen der Hochspannungsmessung über die erste Hilfswicklung kann die zweite Hilfswicklung die Erstfehlersicherheit erhöhen. Die Messungen über die parallelen Hilfswicklungen können auch miteinander abgeglichen werden und bei Abweichungen ggf. ein Fehler angezeigt bzw. eine Wartung ausgelöst werden.

Optional kann die erste und/oder die zweite Hilfswicklung an einer mit Masse verbundenen Erdungsstirnseite des Transformators angeordnet sein. Dies hat den Vorteil, dass die erste und/oder die zweite Hilfswicklung weniger im Magnetfeld des Transformators gespeicherte Energie als Strom aufnimmt, die dann der Hochspannungswicklung zur Ladung der Entladekondensatoren fehlt.

Optional können die Primärwicklung sowie die erste und/oder die zweite Hilfswicklung auf einem Primärspulenformer gewickelt sein. Damit ist das Vorsehen der ersten und/oder die zweiten Hilfswicklung beim Zusammenbau des Transformators besonders einfach.

Optional kann das Abstandsmittel ein Material aufweisen mit einer relativen Permittivität von mehr als 3,0 bei einer Frequenz von 1,0 kHz und einer Temperatur von 23 °C. Es hat sich in Simulationen und Testversuchen gezeigt, dass die maximal auftretenden Feldstärken in diesem Bereich der Permittivität der Abstandsmittel unter den zulässigen Maximalwerten, z.B. 20 kV/mm, für die geforderte Isolationsfestigkeit liegen.

Optional kann die Isolationsmasse ein Material aufweisen mit einer relativen Permittivität von mehr als 4,0 bei einer Frequenz von 1,0 kHz und einer Temperatur von 23 °C. Es hat sich in Simulationen und Testversuchen gezeigt, dass die maximal auftretenden Feldstärken in diesem Bereich der Permittivität der Isolationsmasse unter den zulässigen Maximalwerten, z.B. 30 kV/mm, für die geforderte Isolationsfestigkeit liegen.

Optional kann der Transformator einen Ferritkern aufweisen mit einer Sättigungsflussdichte von mindestens 200 mT. Damit wird eine effiziente Energieübertragung von der Primärwicklung auf die Hochspannungswicklung gewährleistet. Der Ferritkern kann aus verstäubten Metalloxiden mit feiner Körnung hergestellt sein.

Das hierin offenbarte Schallwellenbehandlungsgerät ist nachfolgend anhand von in der Zeichnung dargestellten Ausführungsformen beispielhaft erläutert. Es zeigen:

Fig. 1    eine beispielhafte Ausführungsform des hierin offenbarten Schallwellenbehandlungsgeräts;

Fig. 2    ein Schaltbild einer beispielhaften Ausführungsform der Hochspannungseinheit des hierin offenbarten Schallwellenbehandlungsgeräts;

Fig. 3a   einen Querschnitt einer beispielhaften Ausführungsform des Transformators der Hochspannungseinheit des hierin offenbarten Schallwellenbehandlungsgeräts; und

Fig. 3b     einen Detailausschnitt des Querschnitts einer beispielhaften Ausführungsform des Transformators der Hochspannungseinheit des hierin offenbarten Schallwellenbehandlungsgeräts.

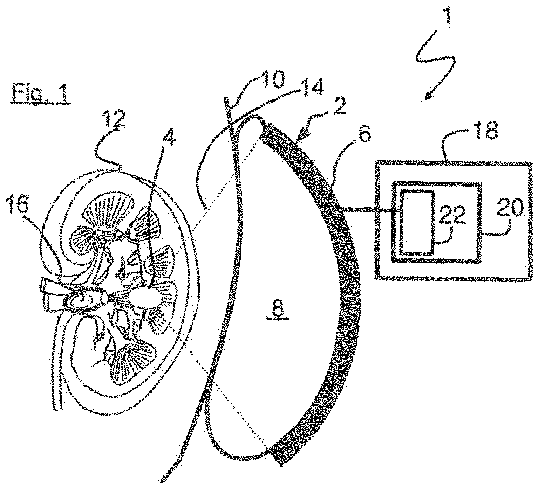

Bei dem in Figur 1 dargestellten Schallwellenbehandlungsgerät 1 handelt es sich um eine Vorrichtung zur Stoßwellenlithotripsie. Diese Vorrichtung weist eine Therapiefeldquelle in Form eines Schallwellenerzeugers 2 auf. Der Schallwellenerzeuger 2 dient zur Fragmentierung eines Nierensteins 4, kann aber auch zur Fragmentierung anderer Körpersteine, wie beispielsweise Blasen- und Harnleitersteinen im Harntrakt eingesetzt werden.

Der Schallwellenerzeuger 2 weist eine Trägerkalotte 6 mit einer Vielzahl darauf befestigter Piezoelemente sowie einen sich an die Trägerkalotte 6 distalseitig anschließenden weichelastischen Koppelbalg 8 auf. Zu Therapiebeginn wird der Schallwellenerzeuger 2 mit dem Koppelbalg 8 an der Körperaußenseite 10 des Patienten in einem an die Niere 12 angrenzenden Bereich angelegt. Anschließend muss sichergestellt werden, dass die Lage des Fokus eines vom Schallwellenerzeuger 2 emittierten fokussierten akustischen Stoßwellenfeldes 14 mit der Lage des zu fragmentierenden Nierensteins 4 übereinstimmt, um die Energie des Stoßwellenfeldes 14 nur in den Nierenstein 4 und nicht in das umgebende Gewebe einzuleiten.

Zur genauen Ausrichtung des Fokus des Stoßwellenfeldes 14 auf den Nierenstein 4 kann eine Vorrichtung zur bildgebenden Erfassung des Behandlungsgebiets, im vorliegenden Fall des Nierensteins 4, vorgesehen sein. Diese Vorrichtung ist in einem Kapselendoskop 16 angeordnet, das intrakorporal in der Niere 12 eingesetzt wird.

Hier nicht gezeigt kann die bildgebende Erfassung des Behandlungsgebiets allerdings auch extrakorporal ermittelt werden, wie zum Beispiel durch geeignete und genaue Ultraschall-Sonographie, Röntgen- bzw. Computertomographie (CT), Magnetresonanz- oder Kernspintomographie (MRT), Positronen-Emissions-Tomographie (PET) oder Kombinationen daraus. Beispielsweise kann im Koppelbalg 8 eine auf den Patienten gerichtete Ultraschall-Sonde angeordnet sein und der Patient mittels eines Röntgen-C-Bogens in einer Achse durchleuchtet werden, die einen Winkel mit der Achse der Ultraschall-Sonde einschließt. Damit könnte dann die Lage des Nierensteins 4 extrakorporal ermittelt werden.

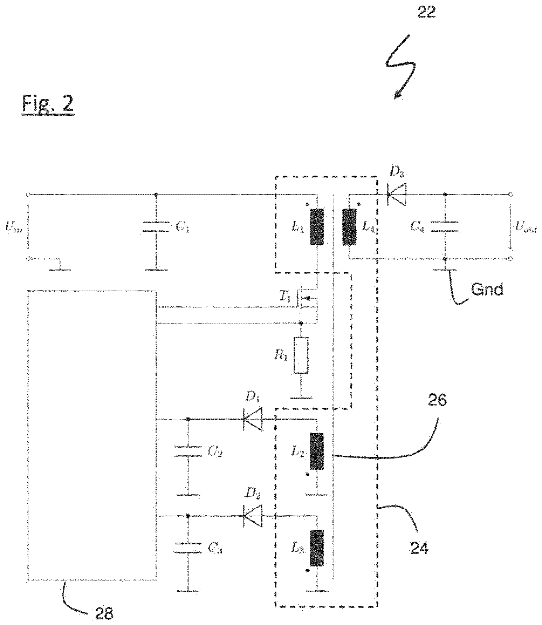

Der Schallwellenerzeuger 2 ist mit einer Steuereinheit 18 verbunden, die eine elektrische Hochspannungseinheit 20 aufweist, welche wiederum eine Sperrwandlereinheit 22 aufweist. Die Hochspannungseinheit 20 ist dazu eingerichtet, die mit dem Schallwellenerzeuger 2 gekoppelten Piezoelemente mit elektrischer Hochspannung zu versorgen. Dies geschieht über Entladekondensatoren (nicht gezeigt), die von oder in der Hochspannungseinheit 20 geladen werden und sich dann beispielsweise mittels Thyristorschaltern konzertiert über die Piezoelemente entladen. Die Piezoelemente wandeln die so erhaltene elektrische Energie zumindest teilweise in Bewegungsenergie bzw. Verformungsenergie um und erzeugen damit im Schallwellenerzeuger 2 das Stoßwellenfeld 14. Die Ladung der Entladekondensatoren erfolgt hierbei über die Sperrwandlereinheit 22 wie in Figur 2 näher beschrieben.

Das in Figur 2 gezeigte Schaltbild zeigt schematisch wie die Sperrwandlereinheit 22 geschaltet ist. Die Sperrwandlereinheit 22 weist einen Transformator 24 auf mit einer eingangsseitigen Primärwicklung $L_1$, einer ersten Hilfswicklung $L_2$, einer zweiten Hilfswicklung $L_3$, und einer ausgangsseitigen Hochspannungswicklung $L_4$, wobei die Wicklungen einen gemeinsamen Ferritkern 26 aufweisen. Über eine Mess-, Regel- und Überwachungseinheit 28, die Teil der Hochspannungseinheit 20 oder Teil der Steuereinheit 18 sein kann, wird die Funktionsweise des Transformators 24 gemessen, geregelt und überwacht. Die Mess-, Regel- und Überwachungseinheit 28 kann dazu einen Transistor $T_1$ leitend schalten und mittels einer Eingangsspannung $U_{in}$ die Primärwicklung $L_1$ bestromen, bis ein über einen Widerstand $R_1$ einstellbarer Maximalstrom erreicht ist. Solange Transistor $T_1$ leitend ist, sperrt eine ausgangsseitig mit der Hochspannungswicklung $L_4$ verbundene Diode $D_3$ einen Stromfluss in der Hochspannungswicklung $L_4$. Ebenso sperren Dioden $D_1$ bzw. $D_2$ Ströme durch die erste Hilfswicklung $L_2$ bzw. die zweite Hilfswicklung $L_3$. Die Wicklungen weisen jeweils entsprechende Kapazitäten $C_1$, $C_2$, $C_3$ und $C_4$ auf, die bei der Dimensionierung und Ausgestaltung des Transformators mit einbezogen werden müssen. Die erste Hilfswicklung $L_2$ und die zweite Hilfswicklung $L_3$ sind vorzugsweise nicht als Drahtwicklungen, sondern als zueinander parallele Folienwicklungen ausgeführt. In Versuchen hat dies eine höhere Regelgenauigkeit gezeigt.

Die Sperrwandlereinheit 22 speichert also während einer Leitphase des Transistors $T_1$ über einen Strom durch die Primärwicklung $L_1$ Energie im Magnetfeld. Sobald der Transistors $T_1$ den Strom durch die Primärwicklung $L_1$ unterbricht (Sperrphase), führt die im Magnetfeld gespeicherte Energie zu einem Strom in der ausgangsseitigen Hochspannungswicklung $L_4$, der ersten Hilfswicklung $L_2$ und der zweiten Hilfswicklung $L_3$ jeweils in Durchlassrichtung der Dioden $D_4$, $D_2$ und $D_3$. Der Strom in der ausgangsseitigen Hochspannungswicklung $L_4$ baut eine ausgangsseitige Hochspannung $U_{out}$ zum Aufladen von (nicht gezeigten) Entladekondensatoren auf, wobei die ausgangsseitige Hochspannung $U_{out}$ unabhängig voneinander mittels der ersten Hilfswicklung $L_2$ und der zweiten Hilfswicklung $L_3$ in der Mess-, Regel- und Überwachungseinheit 28 gemessen werden kann. Die erste Hilfswicklung $L_2$ und die zweite Hilfswicklung $L_3$ sind dabei vorteilhafterweise auf einer mit Masse Gnd verbundenen Ausgangsseite des Transformators 24 angeordnet.

Die Mess-, Regel- und Überwachungseinheit 28 schaltet den Transistor $T_1$ getaktet (mit über 20 kHz bis 10 MHz, also über dem Hörbereich zur Vermeidung von Störgeräuschen und gut simulierbar) zwischen Leitphase und Sperrphase, sodass die Entladekondensatoren in etwa 100 ms bis 200 ms auf eine Hochspannung von beispielsweise 8,9 kV in etwa 5.000 bis 2.000.000 Takten geladen werden können. Die Energie, die von der Primärwicklung $L_1$ auf die Hochspannungswicklung $L_4$ jeweils pro Takt übertragen wird, ist dabei im Magnetfeld zwischengespeichert, vorzugsweise in einem Luftspalt des Ferritkerns 26. Die Hochspannungswicklung $L_4$ ist also galvanisch getrennt von der Mess-, Regel- und Überwachungseinheit 28, was zur Erstfehlersicherheit beiträgt.

Simulationen und praktische Versuche haben gezeigt, dass der Transformator 24 nach einem Hochspannungspuls von etwa 5 μs zum Abbau von Restmagnetisierung und -ladung ca. 5 μs braucht, wodurch sich ein maximales Tastverhältnis $v_T = 0,5$ ergibt. Bei Verwendung eines Ferritkerns mit beispielsweise einer Sättigungsflussdichte $B_{sat} = 350$ mT und einer magnetischen Wirkfläche von $A = 209$ mm$^2$ ergibt sich mit einer Primärwicklung $L_1$ von N = 5 Windungen und einer Taktfrequenz von 50 kHz folgende Eingangswechselspannung $U_{in}$:

$$U_{in} = \frac{N \cdot B_{sat} \cdot A \cdot f}{\vartheta_T} = \frac{5 \cdot 0,35\,T \cdot 209 \cdot 10^{-6}m^2 \cdot 50 \cdot 10^3 s^{-1}}{0,5} = 36,58\,V$$

Wenn die Hochspannungswicklung $L_4$ 85 bis 100 Windungen aufweist, z.B. 95, also der Transformator 24 beispielsweise ein Windungsverhältnis von ca. 18 bis 19 hat, kann eine Hochspannung von 8,9 kV an den Entladekondensatoren in ca. 150 ms aufgebaut werden.

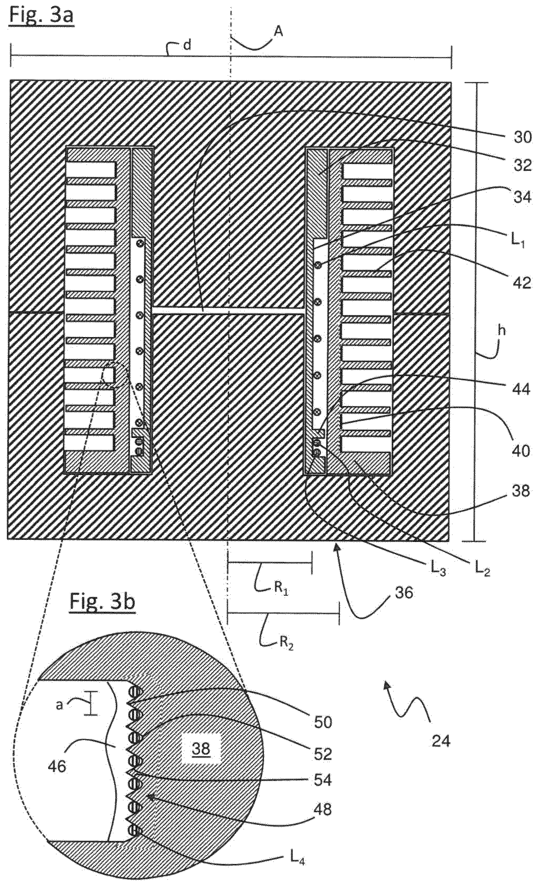

Figur 3a zeigt den geometrischen Aufbau des Transformators 24 im Querschnitt mit einer Längsachse A. Mit den hierin beispielshaft beschriebenen Parametern kann der Transformator 24 mit einem Durchmesser d von weniger als 50 mm und mit einer Höhe h von weniger als 50 mm aufgeführt werden. Der Ferritkern 26 ist zweiteilig ausgestaltet, wobei die beiden Teile einen 0,3 mm bis 1,5 mm, vorzugsweise 1 mm, breiten Luftspalt 30 einschließen. Der Ferritkern 26 kann beispielsweise ein Kern der Form ETD49/25/16 von EPCOS® mit einem sogenannten N87-Ferrit auf MnZn-Basis sein.

Im Ferritkern 26 befindet sich ein innerer Primärspulenformer 32, der eine erste äußere Mantelfläche 34 mit einem ersten Radius $R_1$ definiert. Auf der ersten Mantelfläche 34 ist die Primärwicklung $L_1$ mit fünf Windungen gewickelt. Vorzugsweise besteht dabei die Primärwicklung $L_1$ nicht aus einem Draht, der fünfmal um die Mantelfläche 34 gewickelt ist, sondern aus fünf parallel zueinander geschalteten Drähten, die jeweils einmal um die Mantelfläche 34 gewickelt sind. Dies ist besonders bei hohen Frequenzen von Vorteil, wenn der Strom wegen des Skin-Effekts nicht den Drahtquerschnitt homogen durchfließt, sondern verstärkt auf dem Außenmantel des Drahts fließt. Über den inneren Primärspulenformer 32 ist ein Hochspannungsspulenformer 38 gesteckt, der eine zweite äußere Mantelfläche 40 mit einem zweiten Radius $R_2$ definiert, der größer ist als der erste Radius $R_1$. Vorzugsweise ist der zweite Radius $R_2$ ca. 1 mm bis 3 mm größer als der erste Radius $R_1$, zum Beispiel 1,5 mm. Auf der zweiten Mantelfläche 40 ist die Hochspannungswicklung $L_4$ mit 91 Windungen gewickelt. Der Hochspannungsspulenformer 38 weist 12 Hochspannungsspulenstege 42 auf, die jeweils 13 Abschnitte der Hochspannungswicklung $L_4$ mit jeweils 7 Windungen definieren. Die Primärwicklung $L_1$ befindet sich so vollständig innerhalb der Hochspannungswicklung $L_4$, wobei der Hochspannungsspulenformer 38 den Primärspulenformer 32 koaxial umgreift.

An einer Erdungsstirnseite 36 des Transformators 24 sind die erste Hilfswicklung $L_2$ und die zweite Hilfswicklung $L_3$ auf den Primärspulenformer 32 gewickelt und mittels eines Primärspulenstegs 44 von der Primärwicklung $L_1$ beabstandet und isoliert gehalten. An der Erdungsstirnseite 36, an der sich die erste Hilfswicklung $L_2$ und die zweite Hilfswicklung $L_3$ befinden, sind vorzugsweise die Primärwicklung $L_1$ und die Hochspannungswicklung $L_4$ mit Masse Gnd verbunden. Da der Strom in der Primärwicklung $L_1$ über 20 A betragen kann, ist ein Drahtdurchmesser von 0,5 mm oder mehr für die fünf parallelen Drähte der Primärwicklung $L_1$ vorteilhaft.

Wie in der Detailansicht in Fig. 3b erkennbar, ist die Hochspannungswicklung $L_4$ im Wesentlichen luftblasenfrei in eine Isolationsmasse 46 eingebettet, wobei die Isolationsmasse 46 vorzugsweise unter Vakuumverschluss über die Hochspannungswicklung $L_4$ vergossen und gehärtet wurde. Die 7 Hochspannungswindungen pro Abschnitt der Hochspannungswicklung $L_4$ sind auf ein Feingewinde auf der zweiten äußeren Mantelfläche 40 gewickelt, wobei das Feingewinde als Abstandsmittel 48 fungiert. Benachbarte Hochspannungswindungen innerhalb eines Abschnitts der Hochspannungswicklung $L_4$ haben dadurch einen genau definierten Abstand a voneinander. Gewindestege 50 ragen dabei radial aus dem Hochspannungsspulenformer 38 zwischen benachbarte Hochspannungswindungen, die in einer V-förmigen Gewindenut 52 auf der zweiten äußeren Mantelfläche 40 liegen. Die Gewindestege 50 bzw. die V-förmige Gewindenut 52 definieren Zentrierflächen 54, die die Hochspannungswindungen in eine eindeutige Lage zwingen. Vorzugsweise sind die Hochspannungswindungen mit vollem Umfang in die Gewindenut 52 eingebettet bzw. ragen die Gewindestege 50 über den Drahtdurchmesser der Hochspannungswicklung $L_4$ hinaus zwischen benachbarte Hochspannungswindungen.

Der Hochspannungsspulenformer 38 und damit in dieser Ausführungsform auch das Abstandsmittel 48 sind vorzugsweise aus TECAPEEK® gefertigt, das eine relative Permittivität von 3,3 hat bei einer Frequenz von 1,0 kHz und einer Temperatur von 23 °C. Die Isolationsmasse kann beispielsweise WEVO® PU 552 FL mit einem Härter 300 im Mischungsverhältnis 5:1 sein, die eine relative Permittivität von 4,6 bei einer Frequenz von 1,0 kHz und einer Temperatur von 23 °C aufweist, sodass eine Isolationssicherheit bis zu einer maximalen Feldstärke von 29 kV/mm gewährleistet ist.

Das hierin offenbarte Schallwellenbehandlungsgerät kann isolationsfest und erstfehlersicher mit einem Hochspannungstransformator 24 betrieben werden, der mit einem Durchmesser d von weniger als 50 mm und mit einer Höhe h von weniger als 50 mm ausgeführt werden kann, und weniger als 0,5 kg wiegt. Außerdem stellt der hierin beschriebene Hochspannungstransformator 24 eine Leistung von mindestens 70 W bei einem Wirkungsgrad von mehr als 80% sicher.

**Bezugszeichenliste**

| | |
|---|---|
| 1 | - Schallwellenbehandlungsgerät |
| 2 | - Schallwellenerzeuger |
| 4 | - Nierenstein des Patienten |
| 6 | - Trägerkalotte |
| 8 | - Koppelbalg |
| 10 | - Körperaußenseite des Patienten |
| 12 | - Niere des Patienten |
| 14 | - Stoßwellenfeld |
| 16 | - Kapselendoskop |
| 18 | - Steuereinheit |
| 20 | - Hochspannungseinheit |
| 22 | - Sperrwandlereinheit |
| 24 | - Transformator |
| 26 | - Ferritkern |
| 28 | - Mess-, Regel- und Überwachungseinheit |
| 30 | - Luftspalt |
| 32 | - Primärspulenformer |
| 34 | - erste Mantelfläche |
| 36 | - Erdungsstirnseite |
| 38 | - Hochspannungsspulenformer |
| 40 | - zweite Mantelfläche |
| 42 | - Hochspannungsspulenstege |
| 44 | - Primärspulensteg |

| | |
|---|---|
| 46 | - Isolationsmasse |
| 48 | - Abstandsmittel |
| 50 | - Gewindestege |
| 52 | - Gewindenut |
| 54 | - Zentrierflächen |
| $L_1$ | - Primärwicklung |
| $L_2$ | - erste Hilfswicklung |
| $L_3$ | - zweite Hilfswicklung |
| $L_4$ | - Hochspannungswicklung |
| $D_1$ | - Diode an der ersten Hilfswicklung |
| $D_2$ | - Diode an der zweiten Hilfswicklung |
| $D_3$ | - Diode an der Hochspannungswicklung |
| $C_1$ | - Kapazität an der Primärwicklung |
| $C_2$ | - Kapazität an der ersten Hilfswicklung |
| $C_3$ | - Kapazität an der zweiten Hilfswicklung |
| $C_4$ | - Kapazität an der Hochspannungswicklung |
| $U_{in}$ | - Eingangsspannung |
| $U_{out}$ | - ausgangsseitige Hochspannung |
| $R_1$ | - Widerstand |
| $T_1$ | - Transistor |
| $R_1$ | - erster Radius |
| $R_2$ | - zweiter Radius |
| A | - Längsachse des Transformators |
| Gnd | - Masseanschluss der Hochspannungswicklung |
| a | - Abstand benachbarter Hochspannungswindungen |
| d | - Durchmesser des Transformators |
| h | - Höhe des Transformators |

**Patentansprüche**

1.  Schallwellenbehandlungsgerät (1) für die medizinische Behandlung mit Druckwellen, insbesondere zur Lithotripsie, mit

- einem Schallwellenerzeuger (2),
- einer Mehrzahl von Piezoelementen, die mit dem Schallwellenerzeuger (2) gekoppelt sind, und
- einer elektrischen Hochspannungseinheit (20), die dazu eingerichtet ist, die Piezoelemente mit elektrischer Hochspannung zu versorgen,

**dadurch gekennzeichnet, dass**
die Hochspannungseinheit (20) eine Sperrwandlereinheit (22) mit einem Transformator (24) aufweist, wobei der Transformator (24) eine Primärwicklung ($L_1$), eine Hochspannungswicklung ($L_4$) und Abstandsmittel (48) aufweist, wobei die Hochspannungswicklung ($L_4$) eine Mehrzahl von Hochspannungswindungen aufweist, die in eine Isolationsmasse (46) eingebettet sind und durch die Abstandsmittel (48) derart positioniert sind, dass benachbarte Hochspannungswindungen einen durch die Abstandsmittel (48) definierten Abstand (a) voneinander haben.

2. Schallwellenbehandlungsgerät nach Anspruch 1, wobei die Hochspannungswindungen im Wesentlichen luftblasenfrei in die Isolationsmasse (46) eingebettet sind.

3. Schallwellenbehandlungsgerät nach Anspruch 1 oder 2, wobei die Hochspannungswicklung ($L_4$) auf einem Hochspannungsspulenformer (38) gewickelt ist und die Abstandsmittel (48) radial aus dem Hochspannungsspulenformer (38) zwischen benachbarte Hochspannungswindungen ragen.

4. Schallwellenbehandlungsgerät nach einem der vorhergehenden Ansprüche, wobei die Hochspannungswicklung ($L_4$) auf einem Hochspannungsspulenformer (38) gewickelt ist und die Hochspannungswindungen in einer auf einer äußeren Mantelfläche (40) des Hochspannungsspulenformers (38) verlaufenden Nut (52) verlaufen.

5. Schallwellenbehandlungsgerät nach Anspruch 3 oder 4, wobei die Abstandsmittel (48) und/oder die Nut (52) Zentrierflächen (54) aufweisen, die die Hochspannungswindungen in eine eindeutige Lage zwingt.

6. Schallwellenbehandlungsgerät nach einem der vorhergehenden Ansprüche, wobei die Hochspannungswicklung ($L_4$) auf einen Hochspannungsspulenformer (38) gewickelt ist und das Abstandsmittel (48) ein Gewinde auf dem Hochspannungsspulenformer (38) ist.

7. Schallwellenbehandlungsgerät nach einem der vorhergehenden Ansprüche, wobei der Transformator (24) eine erste Mantelfläche (34) mit einem ersten Radius ($R_1$) und eine zweite Mantelfläche (40) mit einem zweiten Radius ($R_2$) aufweist, wobei die Primärwicklung ($L_1$) auf der ersten Mantelfläche (34) und die Hochspannungswicklung ($L_4$) auf der zweiten Mantelfläche (40) gewickelt ist, wobei der erste Radius ($R_1$) anders, vorzugsweise kleiner, ist als der zweite Radius ($R_2$).

8. Schallwellenbehandlungsgerät nach einem der vorhergehenden Ansprüche, wobei die Primärwicklung ($L_1$) zumindest teilweise innerhalb der Hochspannungswicklung ($L_4$) verläuft.

9. Schallwellenbehandlungsgerät nach einem der vorhergehenden Ansprüche, wobei die Hochspannungswicklung ($L_4$) auf einem Hochspannungsspulenformer (38) und die Primärwicklung ($L_1$) auf einem Primärspulenformer (32) gewickelt ist, wobei der Hochspannungsspulenformer (38) den Primärspulenformer (32) koaxial umgreift.

10. Schallwellenbehandlungsgerät nach einem der vorhergehenden Ansprüche, wobei der Transformator (24) eine erste Hilfswicklung ($L_2$) zur indirekten Messung der ausgangsseitigen Hochspannung aufweist.

11. Schallwellenbehandlungsgerät nach Anspruch 10, wobei der Transformator (24) eine zweite Hilfswicklung ($L_3$) zur redundanten indirekten Messung der ausgangsseitigen Hochspannung aufweist, die parallel zur ersten Hilfswicklung ($L_2$) angeordnet und geschaltet ist.

12. Schallwellenbehandlungsgerät nach Anspruch 10 oder 11, wobei die erste und/oder die zweite Hilfswicklung ($L_2$, $L_3$) an einer mit Masse (Gnd) verbundenen Erdungsstirnseite (36) des Transformators (24) angeordnet ist.

13. Schallwellenbehandlungsgerät nach einem der Ansprüche 10 bis 11, wobei die Primärwicklung ($L_1$) sowie die erste und/oder die zweite Hilfswicklung ($L_2$, $L_3$) auf einem Primärspulenformer (32) gewickelt sind.

14. Schallwellenbehandlungsgerät nach einem der vorhergehenden Ansprüche, wobei das Abstandsmittel (48) ein Material aufweist mit einer relativen Permittivität von mehr als 3,0 bei einer Frequenz von 1,0 kHz und einer Temperatur von 23 °C.

15. Schallwellenbehandlungsgerät nach einem der vorhergehenden Ansprüche, wobei die Isolationsmasse (46) ein Material aufweist mit einer relativen Permittivität von mehr als 4,0 bei einer Frequenz von 1,0 kHz und einer Temperatur von 23 °C.

16. Schallwellenbehandlungsgerät nach einem der vorhergehenden Ansprüche, wobei der Transformator (24) einen Ferritkern (26) aufweist mit einer Sättigungsflussdichte von mindestens 200 mT.


**Claims**

1. A sound wave treatment device (1) for medical treatment using compression waves, in particular for lithotripsy, having:

   - a sound wave generator (2),
   - a plurality of piezo elements that are coupled to the sound wave generator (2), and,
   - an electrical high voltage unit (20) that is set up for supplying the piezo elements with high electrical voltage

   **characterized in that**
   the high voltage unit (20) has a blocking converter unit (22) having a transformer (24), wherein the transformer (24) has a primary coil ($L_1$), a high voltage coil ($L_4$), and spacers (48), wherein the high voltage coil ($L_4$) has a plurality of high voltage windings that are embedded in an insulating mass (46) and are positioned using the spacers (48) such that adjacent high voltage windings are at a defined distance (a) from one another due to the spacers (48).

2. The sound wave treatment device according to claim 1, wherein the high voltage windings are embedded in the insulating mass (46) essentially free of air bubbles.

3. The sound wave treatment device according to claim 1 or 2, wherein the high voltage coil ($L_4$) is wound on a high voltage coil former (38) and the spacers (48) project radially out of the high voltage coil former (38) between adjacent high voltage windings.

4. The sound wave treatment device according to any of the preceding claims, wherein the high voltage coil ($L_4$) is wound on a high voltage coil former (38) and the high voltage windings run in a groove (52) running on an outer curved surface area (40) of the high voltage coil former (38).

5. The sound wave treatment device according to claim 3 or 4, wherein the spacers (48) and/or the groove (52) have centering surfaces (54) that urge the high voltage windings into a specific position.

6. The sound wave treatment device according to any of the preceding claims, wherein the high voltage coil ($L_4$) is wound on a high voltage coil transformer (38) and the spacers (48) are a thread on the high voltage coil transformer (38).

7. The sound wave treatment device according to any of the preceding claims, wherein the transformer (24) has a first curved surface area (34) having a first radius ($R_1$) and a second curved surface area (40) having a second radius ($R_2$), wherein the primary coil ($L_1$) is wound on the first curved surface area (34) and the high voltage coil ($L_4$) is wound on the second curved surface area (40), wherein the first radius ($R_1$) is different, preferably smaller, than the second radius ($R_2$).

8. The sound wave treatment device according to any of the preceding claims, wherein the primary coil ($L_1$) runs, at least in part, within the high voltage coil ($L_4$).

9. The sound wave treatment device according to any of the preceding claims, wherein the high voltage coil ($L_4$) is wound on a high voltage coil former (38) and the primary coil ($L_1$) is wound on a primary coil former (32), wherein the high voltage coil former (38) coaxially surrounds the primary coil former (32).

10. The sound wave treatment device according to any of the preceding claims, wherein the transformer (24) has a first

auxiliary coil ($L_2$) for indirectly measuring the output-side high voltage.

11. The sound wave treatment device according to claim 10, wherein the transformer (24) has a second auxiliary coil ($L_3$) that is for redundant indirect measurement of the output-side high voltage and that is arranged and switched parallel to the first auxiliary coil ($L_2$).

12. The sound wave treatment device according to claim 10 or 11, wherein the first and/or the second auxiliary coil ($L_2$, $L_3$) are arranged on a ground end face (36), connected to a ground (Gnd), of the transformer (24).

13. The sound wave treatment device according to any of claims 10 through 11, wherein the primary coil ($L_1$) and the first and/or the second auxiliary coil ($L_2$, $L_3$) are wound on a primary coil former (32).

14. The sound wave treatment device according to any of the preceding claims, wherein the spacers (48) have a material having a relative permittivity of greater than 3.0 at a frequency of 1.0 kHz and a temperature of 23 °C.

15. The sound wave treatment device according to any of the preceding claims, wherein the insulating mass (46) has a material having a relative permittivity of greater than 4.0 at a frequency of 1.0 kHz and a temperature of 23 °C.

16. The sound wave treatment device according to any of the preceding claims, wherein the transformer (24) has a ferrite core (26) having a saturation flux density of at least 200 mT.


**Revendications**

1. Appareil de traitement aux ondes sonores (1) destiné au traitement médical par des ondes de pression, en particulier pour la lithotripsie, comportant :

   - un générateur d'ondes sonores (2),
   - plusieurs éléments piézoélectriques qui sont couplés avec le générateur d'ondes sonores (2), et
   - une unité électrique haute tension (20) qui est montée pour alimenter en haute tension électrique les éléments piézoélectriques,

   **caractérisé en ce que** l'unité haute tension (20) présente une unité de convertisseur flyback (22) avec transformateur (24), le transformateur (24) présentant un enroulement primaire ($L_1$), un enroulement haute tension ($L_4$) et des dispositifs d'écartement (48), l'enroulement haute tension ($L_4$) présentant plusieurs enroulements haute tension qui sont incorporés dans une masse d'isolation (46) et qui sont positionnés de façon telle via les dispositifs d'écartement (48) que des enroulements haute tension adjacents présentent un écart (a) aux autres défini par les dispositifs d'écartement (48).

2. Appareil de traitement aux ondes sonores selon la revendication 1, dans lequel les enroulements haute tension sont incorporés dans la masse d'isolation (46), essentiellement sans bulles d'air.

3. Appareil de traitement aux ondes sonores selon la revendication 1 ou 2, dans lequel l'enroulement haute tension ($L_4$) est enroulé sur un dispositif de bobinage haute tension (38) et les dispositifs d'écartement (48) font saillie radialement depuis le dispositif de bobinage haute tension (38) entre des enroulements haute tension adjacents.

4. Appareil de traitement aux ondes sonores selon l'une des revendications précédentes, dans lequel l'enroulement haute tension ($L_4$) est enroulé sur un dispositif de bobinage haute tension (38) et les enroulements haute tension s'étendent dans une rainure (52) qui s'étend sur une surface d'enveloppe externe (40) du dispositif de bobinage haute tension (38).

5. Appareil de traitement aux ondes sonores selon la revendication 3 ou 4, dans lequel les dispositifs d'écartement (48) et/ou la rainure (52) présentent des surfaces de centrage (54) qui contraignent les enroulements haute tension dans une position univoque.

6. Appareil de traitement aux ondes sonores selon l'une des revendications précédentes, dans lequel l'enroulement haute tension ($L_4$) est enroulé sur un dispositif de bobinage haute tension (38) et le dispositif d'écartement (48) est un filetage sur le dispositif de bobinage haute tension (38).

7. Appareil de traitement aux ondes sonores selon l'une des revendications précédentes, dans lequel le transformateur (24) présente une première surface d'enveloppe (34) de premier rayon ($R_1$) et une deuxième surface d'enveloppe (40) de deuxième rayon ($R_2$), l'enroulement primaire ($L_1$) étant enroulé sur la première surface d'enveloppe (34) et l'enroulement haute tension ($L_4$) sur la deuxième surface d'enveloppe (40), le premier rayon ($R_1$) étant différent du deuxième rayon ($R_2$), de préférence plus petit.

8. Appareil de traitement aux ondes sonores selon l'une des revendications précédentes, dans lequel l'enroulement primaire ($L_1$) s'étend au moins partiellement à l'intérieur de l'enroulement haute tension ($L_4$).

9. Appareil de traitement aux ondes sonores selon l'une des revendications précédentes, dans lequel l'enroulement haute tension ($L_4$) est enroulé sur un dispositif de bobinage haute tension (38) et l'enroulement primaire ($L_1$) sur un dispositif de bobinage primaire (32), le dispositif de bobinage haute tension (38) entourant le dispositif de bobinage primaire (32) de façon coaxiale.

10. Appareil de traitement aux ondes sonores selon l'une des revendications précédentes, dans lequel le transformateur (24) présente un premier enroulement auxiliaire ($L_2$) permettant la mesure indirecte de la haute tension côté sortie.

11. Appareil de traitement aux ondes sonores selon la revendication 10, dans lequel le transformateur (24) présente un second enroulement auxiliaire ($L_3$) destiné à la mesure indirecte redondante de la haute tension côté sortie et qui est disposé et commuté parallèlement au premier enroulement auxiliaire ($L_2$).

12. Appareil de traitement aux ondes sonores selon la revendication 10 ou 11, dans lequel le premier et/ou le deuxième enroulements) auxiliaire(s) ($L_2$, $L_3$) est/sont disposé(s) au niveau d'une face avant de point de masse (36) du transformateur (24) reliée à une masse (Gnd).

13. Appareil de traitement aux ondes sonores selon l'une des revendications 10 à 11, dans lequel l'enroulement primaire ($L_1$) ainsi que le premier et/ou le deuxième enroulement(s) auxiliaire(s) ($L_2$, $L_3$) sont enroulés sur un dispositif de bobinage primaire (32).

14. Appareil de traitement aux ondes sonores selon l'une des revendications précédentes, dans lequel le dispositif d'écartement (48) présente un matériau qui a une permittivité relative supérieure à 3,0 à une fréquence de 1,0 kHz et une température de 23 °C.

15. Appareil de traitement aux ondes sonores selon l'une des revendications précédentes, dans lequel la masse isolante (46) présente un matériau qui a une permittivité relative supérieure à 4,0 à une fréquence de 1,0 kHz et une température de 23 °C.

16. Appareil de traitement aux ondes sonores selon l'une des revendications précédentes, dans lequel le transformateur (24) présente un noyau de ferrite (26) ayant une densité de flux à saturation d'au moins 200 mT.

Fig. 1

Fig. 2

## Fig. 3a

## Fig. 3b

EP 3 424 445 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010055836 B4
- DE 4433224 C1
- EP 1163884 A1
- US 4408176 A
- US 20120203297 A1